# EUROPEAN PATENT APPLICATION

(11) **EP 4 130 290 A1**
(43) Date of publication of application: **08.02.2023**
(21) Application number: 21775964.6
(22) Date of filing: 28.01.2021
(51) Int. Cl.: C12Q 1/68

(54) **APPLICATION OF RED BLOOD CELL NUCLEIC ACID IN IDENTIFYING TUMOR MUTATION TYPES**

(30) Priority: 23.03.2020 CN 202010208852
(71) Applicant: My-Biomed Technology (Guangzhou) Co., Ltd., Guangzhou, Guangdong 510530 (CN)
(72) Inventor: CHEN, Qihan, Nanjing, Jiangsu 210032 (CN); ZHU, Weiyan, Nanjing, Jiangsu 210032 (CN); TONG, Yanzheng, Nanjing, Jiangsu 210032 (CN)
(74) Representative: Impact Intellectual Property LLP
(86) International application number: PCT/CN2021/074035
(87) International publication number: WO 2021/190124

(57) **Abstract**

Provided are a method and corresponding kit for diagnosing tumors by detecting mutations in red blood cell nucleic acid. The method and kit in the present invention can accurately identify tumor formation and/or mutation types so as to accurately diagnose tumors.

## Description

### CROSS REFERENCE TO RELATED APPLICATIONS

The present application claims priority to Chinese Patent Application No. 202010208852.5, filed on March 23, 2020, entitled "Application of Red blood cell Nucleic Acid in Identifying Tumor Mutation Types" which is incorporated by reference herein in its entirety.

### FIELD OF THE INVENTION

The present invention relates to the field of bioassays and, more specifically, to the application of red blood cell nucleic acid in identification of tumorigenesis and/or mutation type of tumor.

### BACKGROUND OF THE INVENTION

With the significant decrease in the cost of genetic diagnosis, genetic diagnosis technology is gradually entering the clinic. The information from genetic diagnosis is increasingly being used clinically for precision medicine. As a next-generation treatment technology, precision medicine has great technical advantages over traditional treatment methods. Compared with traditional treatment methods, precision medicine has the advantages of accuracy and convenience. For example, gene sequencing can identify the mutated genes of cancer, so that the right drugs can be identified quickly, saving patients the time to try various treatments and improving the treatment effect. Samples for genetic diagnosis are mainly from tissues and body fluids (including blood). Although tumor tissue biopsy is the current gold standard for cancer diagnosis and an important tool for cancer treatment, it has become clear in recent years that information obtained from individual biopsy tissues provides a spatially and temporally limited snapshot of the tumor and often does not reflect the heterogeneity of the disease. In addition, the invasive nature of tumor biopsy imposes limitations on repeat sampling.

Liquid biopsy is considered a promising tool for noninvasive tumor analysis and monitoring. For decades, blood has been a rich source of tumor-associated genes, comprising: 1) monocyte fractions: including leukocytes, circulating tumor cells (CTCs) and circulating endothelial cells (CECs); 2) plasma and serum: including extracellular vesicles (EVs), extracellular free DNA (cfDNA) and free RNA (cfRNA), plasma proteins and metabolites, and tumor-educated platelets (TEPs). These biomolecules and biological resources are thought to be part of the tumorigenic and systemic activity of primary tumors (e.g. CTC, CEC, EV, cfRNA, and TEP) or are thought to originate only from passive release of tumor cells during apoptosis and necrosis (e.g. cfDNA) and are mostly in the plasma, or derived from leukocytes. However, on the one hand, the objects of the above liquid biopsy technique are very low in blood; on the other hand, only a very small amount of cfDNA is derived from cancer cells, and most of it is derived from immune cells and other normal cells, resulting in low sensitivity and accuracy of liquid biopsy.

Mutation detection based on existing liquid biopsy technology is mainly used for molecular classification of tumors and clinical drug use guidance. However, due to these technical limitations, it is not possible to provide a highly sensitive test to meet clinical needs, which is particularly difficult in the early stages of tumorigenesis and recurrence. In addition, serum-derived cfDNA has mutations from non-tumor apoptotic and immune cells and cannot be used for differential diagnosis of tumors.

Therefore, there is an urgent need to develop new tumor-specific mutation detection technologies that are simple to operate, rich in sources, and have the sensitivity and accuracy to meet clinical needs.

### Summary of the Invention

It is an object of the present invention to provide a kit and method for identifying tumorigenesis and/or mutation type of tumor.

In order to achieve the above object, the present invention provides the following technical solutions.

A first aspect of the present invention provides a kit, comprising a nucleic acid detection reagent set which is configured to detect a target nucleic acid in red blood cell.

In another preferred embodiment, said kit is configured to identify tumorigenesis and/or mutation type of tumor based on result of target nucleic acid detection in red blood cell.

In another preferred embodiment, said kit is configured to
(1) guide clinical administration of a drug based on identified mutation type of tumor; or
(2) determine molecular classification of tumor of a subject to be tested based on the identified mutation type of the tumor; or
(3) be used for positive differential diagnosis of tumor based on identified mutation.

In another preferred embodiment, said nucleic acid detection reagent set comprises a red blood cell lysis reagent.

In another preferred embodiment, said nucleic acid detection reagent set further comprises a red blood cell isolation reagent.

In another preferred embodiment, said nucleic acid detection reagent set further comprises a nucleic acid extraction reagent.

In another preferred embodiment, said nucleic acid extraction reagent is a DNA extraction reagent or an RNA extraction reagent.

In another preferred embodiment, said target nucleic acid is a tumor-specific gene mutation.

In another preferred embodiment, said target nucleic acid is derived from a tumor cell.

In another preferred embodiment, said reagent set comprises a probe and/or a primer specific for the target nucleic acid.

In another preferred embodiment, said reagent set is suitable for one or more of the following techniques or methods: PCR technology, crisper/cas technology (e.g. crisper/cas12 technology), immunofluorescence assay, high-throughput sequencing (NGS) technology.

In another preferred embodiment, said reagent set comprises a primer for specific amplification of said target nucleic acid.

In another preferred embodiment, said kit further comprises an instruction which states that said kit is for detection of nucleic acid in red blood cell and states that said kit is for identification of mutation type of tumor.

In another preferred embodiment, said tumor is a malignant tumor.

In another preferred embodiment, said malignancy is a malignant solid tumor or a hematological tumor.

In another preferred embodiment, said malignant solid tumor is one or more selected from the group consisting of: lung cancer, breast cancer, esophageal cancer, gastric cancer, thyroid cancer, liver cancer, pancreatic cancer, prostate cancer, cervical cancer, and colorectal cancer.

In another preferred embodiment, a method of using said kit comprises steps of
(a) providing an erythrocyte-derived nucleic acid sample of a subject to be tested; and
(b) detecting one or more tumor-specific gene mutations in the nucleic acid sample.

In another preferred embodiment, gene sequence of target nucleic acid is detected in step (b) to obtain sequence information of the target nucleic acid.

In another preferred embodiment, said method of using said kit further comprises step of (c) determining mutation type of tumor based on the sequence information of the target nucleic acid detected in step (b).

In another preferred embodiment, said target nucleic acid is derived from a tumor cell. For example, a target nucleic acid fragment in the tumor cell enters red blood cell via some pathway and is thus detected within the red blood cell.

A second aspect of the present invention provides a nucleic acid sample for identifying mutation type of tumor or for a positive differential diagnosis of tumor (for identifying occurrence of tumor), and said nucleic acid sample is an erythrocyte-derived nucleic acid.

In another preferred embodiment, said nucleic acid is DNA and/or RNA.

In another preferred embodiment, said nucleic acid is nucleic acid produced by lysis of red blood cells and then extracted.

In another preferred embodiment, said nucleic acid sample is a nucleic acid sample from a tumor-bearing patient.

In another preferred embodiment, said nucleic acid sample comprises nucleic acid derived from a tumor cell.

In another preferred embodiment, the nucleic acid in said nucleic acid sample comprises a tumor-specific gene mutation.

A third aspect of the present invention provides a method for preparing a nucleic acid sample, said nucleic acid sample is used to identify mutation type of tumor, and said method comprising steps of:
(1) isolating red blood cells from whole blood;
(2) extracting nucleic acid from the red blood cells obtained in step (1), thereby producing said nucleic acid sample.

In a further preferred embodiment, said step (1) comprises:
(1a) centrifuging whole blood after anticoagulation, and removing plasma to obtain an erythrocyte-containing precipitate;
(1b) treating the erythrocyte-containing precipitate obtained in step (1a) with lymphocyte isolate, centrifuging to remove nucleated cells to obtain red blood cells.

In another preferred embodiment, in said step (2), the red blood cells obtained in step (1) are lysed using red blood cell lysis buffer, supernatant is taken after centrifugation and nucleic acid is extracted from the supernatant, thereby producing said nucleic acid sample.

A fourth aspect of the present invention provides use of nucleic acid detection reagent set in manufacture of a kit which is used to identify tumorigenesis and/or mutation type of tumor, and said nucleic acid detection set is configured to detect nucleic acid in red blood cell.

In another preferred embodiment, said detection comprises steps of:
(a) preparing an erythrocyte-derived nucleic acid sample of a subject to be tested; and
(b) detecting one or more tumor-specific gene mutations in the nucleic acid sample.

In another preferred embodiment, gene sequence of target nucleic acid is detected in step (b) to obtain sequence information of the target nucleic acid.

In another preferred embodiment, said method of using said kit further comprises steps of determining tumorigenesis and/or mutation type of tumor based on the sequence information of the target nucleic acid detected in step (b).

In another preferred embodiment, said nucleic acid is DNA and/or RNA.

In another preferred embodiment, said nucleic acid is nucleic acid produced by lysis of red blood cells and then extracted.

In another preferred embodiment, said nucleic acid sample is a nucleic acid sample from a tumor-bearing patient.

In another preferred embodiment, said nucleic acid sample comprises nucleic acid derived from a tumor cell.

In another preferred embodiment, the nucleic acid in said nucleic acid sample comprises a tumor-specific gene mutation.

In another preferred embodiment, said tumor is selected from the group consisting of: lung cancer, breast cancer, esophageal cancer, stomach cancer, thyroid cancer, liver cancer, pancreatic cancer, prostate cancer, cervical cancer, and colorectal cancer.

In another preferred embodiment, said nucleic acid detection reagent set comprises a red blood cell lysis reagent.

In another preferred embodiment, said nucleic acid detection reagent set further comprises a red blood cell isolation reagent.

In another preferred embodiment, said nucleic acid detection reagent set further comprises a nucleic acid extraction reagent.

In another preferred embodiment, said nucleic acid extraction reagent is a DNA extraction reagent or an RNA extraction reagent.

In another preferred embodiment, said target nucleic acid is a tumor-specific gene mutation.

In another preferred embodiment, said target nucleic acid is derived from a tumor cell.

In another preferred embodiment, said reagent set comprises a probe and/or a primer specific for the target nucleic acid.

In another preferred embodiment, said detection is accomplished by high-throughput sequencing technology.

In another preferred embodiment, said detection is done by PCR technique.

In another preferred embodiment, said detection is done by the crisper/cas12 technique.

In another preferred embodiment, said detection is accomplished by immunofluorescence.

In another preferred embodiment, said tumor-specific gene mutation comprises a mutation occurring in a gene selected from the group consisting of
EGFR, KRAS, NRAS, NTRK, BRAF, HER2, MET, ALK, ROS-1, RET, BRAC1/2, PTEN, TP53, PIK3CA, RHBDF2, BLM, PALB2, CTHRC1, ASCC1, MSR1, ALDH2, ADH1B, CYP2E1, and GSTM1.

A fifth aspect of the present invention provides a method for identifying tumorigenesis and/or mutation type of tumor, said method comprising steps of:
(a) providing an erythrocyte-derived nucleic acid sample of a subject to be tested; and
(b) detecting one or more tumor-specific gene mutations in the nucleic acid sample.

In another preferred embodiment, gene sequence of target nucleic acid is detected in step (b) to obtain sequence information of the target nucleic acid, and then tumorigenesis and/or mutation type of tumor is determined based on the sequence information detected in step (b).

In another preferred embodiment, said method of using said kit further comprises step of (c) determining tumorigenesis and/or mutation type of tumor based on the sequence information of the target nucleic acid detected in step (b).

In another preferred embodiment, said tumor is selected from the group consisting of: lung cancer, breast cancer, esophageal cancer, stomach cancer, thyroid cancer, liver cancer, pancreatic cancer, prostate cancer, cervical cancer, and colorectal cancer.

In another preferred embodiment, said detection is accomplished by high-throughput sequencing technology.

In another preferred embodiment, said detection is done by PCR technique.

In another preferred embodiment, said detection is done by the crisper/cas12 technique.

In another preferred embodiment, said detection is accomplished by immunofluorescence.

In another preferred embodiment, said tumor-specific gene mutation comprises a mutation occurring in a gene selected from the group consisting of
EGFR, KRAS, NRAS, NTRK, BRAF, HER2, MET, ALK, ROS-1, RET, BRAC1/2, PTEN, TP53, PIK3CA, RHBDF2, BLM, PALB2, CTHRC1, ASCC1, MSR1, ALDH2, ADH1B, CYP2E1, and GSTM1.

It should be understood that, within the scope of the present invention, each of the above technical features of the present invention and each of the technical features specifically described in the following (e.g., embodiments) can be combined with each other so as to constitute new or preferred technical solutions. For the sake of space, they will not be repeated herein.

### Detailed Description of the Invention

Currently, the available literatures in prior art indicate that red blood cells, as anucleated cells, do not possess DNA or cannot synthesize any RNA, and the terms "red blood cell", "erythrocyte", "mature red blood cell" and "mature erythrocyte" used herein are used interchangeably. And the present inventors, after long-term intensive basic research and clinical practice, unexpectedly discovered that nucleic acid samples from red blood cells of tumor patients contained tumor-specific gene mutation. Based on this, the inventors have developed a kit and a method for identifying mutation type of a tumor. The tumor includes lung cancer, breast cancer, esophageal cancer, stomach cancer, thyroid cancer, liver cancer, pancreatic cancer, prostate cancer, cervical cancer, colorectal cancer and other cancers. The present invention has important applications in identification of tumorigenesis and/or mutation type of tumor.

### Terms

### Target Nucleic Acid

As used herein, the term "target nucleic acid" refers to nucleic acid that an examiner (usually a medical examiner) plans to detect, for example, a tumor-specific gene mutation common in the art.

### Gene mutation

As used herein, the term "gene mutation" or "mutation" includes rearrangements, deletions, translocations or fusions of gene fragments, as well as nucleotide substitutions.

### Positive differential diagnosis

As used herein, the terms "positive differential diagnosis" or "positive differential diagnosis for tumor" or "identification of tumorigenesis" refer to confirmation of tumor presence by detection of tumor-specific gene mutation, and/or determination of tumor recurrence by tumor-specific gene mutation detection in patients followed up after tumor treatment and simultaneous updating of tumor-specific gene mutation, etc.

### People to be differentially diagnosed for positive

As used herein, the term "people to be differentially diagnosed for positive " includes tumor-bearing patients to be treated or people at high risk for tumors and/or people who need to be followed up after tumor treatment.

In the present invention, tumor-specific gene mutation includes, but is not limited to, one or a plurality of gene mutations selected from the group consisting of.

| Gene | Mutation name | mutation site | Base changes | Cosmic ID |
|---|---|---|---|---|
| EGFR | EGFR | G719A | 2156G>C | 6239 |
| | Mutation in Exon 18 | G719S | 2155G>A | 6252 |
| | | G719C | 2155G>T | 6253 |
| | EGFR | E746_A750dle (1) | 2235_2249de115 | 6223 |
| | Deletion in Exon 19 (19del) | E746_A750dle (2) | 2236_2250de115 | 6225 |
| | | L747_P753>S | 2240_2257de118 | 12370 |
| | | E746_T751del | 2236_2253de118 | 12728 |
| | | E746_T751>A | 2237_2251de115 | 12678 |
| | | E746_S752>A | 2237_2254de118 | 12367 |
| | | E746_S752>D | 2238_2255de118 | 6220 |
| | | L747_E749del | 2239_2247de19 | 6218 |
| | | L747_T751del | 2239_2253de115 | 6254 |
| | | L747_S752del | 2239_2256de118 | 6255 |
| | | L747_T751>S | 2240_2251de112 | 6210 |
| | | L747_T751del | 2240_2254de115 | 12369 |
| | EGFR | E746_T751>I | 2235_2252>AAT(complex) | 13551 |
| | Other mutations in Exon 19 | E746 S752V | 2237_2255>T(complex) | 12384 |
| | | L747_A750>P | 2238_2248>GC(complex) | 12422 |
| | | L747_T751>Q | 2238_2252>GCA(complex) | 12419 |
| | | L747_A750>P | 2239_2248TTAAGAGAAG>C(complex) | 12382 |
| | | L747_P753>Q | 2239 2258>CA(complex) | 12387 |
| | | L747_T751>P | 2239_2251>C(complex) | 12383 |
| | EGFR | T790M | 2369C>T | 6240 |
| | Mutation in Exon 20 | S768I | 2303G>T | 6241 |
| | | H773_V774insH | 2319_2320insCAC | 12377 |
| | | D770_N771insG | 2310_2311insGGT | 12378 |
| | | V769_D770insASV | 2307_2308insgccagcgtg | 12376 |
| | EGFR | L858R | 2673 T>G | 6224 |
| | Mutation in Exon 21 | L861Q | 2582T>A | 6213 |
| KRAS | KRAS | G12D | 35G>A | 521 |
| | Mutation in Exon 2 | G12A | 35G>C | 522 |
| | | G12V | 35G>T | 520 |
| | | G12S | 34G>A | 517 |
| | | G12C | 34G>T | 516 |
| | KRAS | Q61H | 183A>C | 554 |
| | Mutation in Exon 3 | | | |
| NRAS | NRAS | G12D | 35G>A | 564 |
| | Mutation in Exon 2 | | | |
| | NRAS | Q61R | 182A>G | 584 |
| | Mutation in Exon 3 | | | |
| | NRAS | Q61K | 181C>A | 580 |
| | Mutation in Exon 3 | | | |
| NTRK | K732T | | 2195A>C | 3387125 |
| | R721C | | 2161C>T | 3505000 |
| | R731Q | | 2192G>A | 88824 |
| | P120H | | 359C>A | 1517933 |
| | D576N | | 1726G>A | 222302 |
| BRAF | BRAF | V600E | 1799T>A | 476 |
| | Mutation in Exon 15 | | | |
| HER2 | HER2 | A775_G776insYVMA | 2324_2325ins 12^{∗} | 20959 |

| | | | | |
|---|---|---|---|---|
| | Mutation in Exon 20 | | | |
| MET | MET Intron 14 | | 3082+1 G>T | 24687 |
| ALK | EML4-ALK Fusion mutation | | EML4 exon 13;ALK exon 20 | 463 |
| | | | EML4 exon 6;ALK exon 20 | 474 |
| | | | EML4 exon 20;ALK exon 20 | 465 |
| ROS-1 | CD74-ROS-1 Fusion mutation | | CD74 exon6;ROS1 exon 34 | 1201 |
| | GOPC-ROS-1 Fusion mutation | | GOPC exon 8;ROS1 exon 35 | 1251 |
| RET | KIF5B-RET Fusion mutation | | KIF5B exon 15;RET exon 12 | 1233 |
| BRAC1 | P871L | | 2612C>T | 3755564 |
| | K1183R | | 3548A>G | 148277 |
| | S1613G | | 4837A>G | 3755560 |
| | K654Sfs*47 Frameshift mutation | | 1961del | 1640708 |
| | H619N | | 1855C>A | 4593792 |
| BRAC2 | N372H | | 1114A>C | 147663 |
| | 13033Nfs^{∗}11 Fragment insertion, Frameshift mutation | | 9097dup | 2071558 |
| | N1784Tfs^{∗}7 Deletion, Frameshift mutation | | 5351del | 18607 |
| | R2842C | | 8524C>T | 23938 |
| | R2645Nfs^{∗}3 Deletion, Frameshift mutation | | 7934del | 1738242 |
| PTEN | R130G | | 388C>G | 5219 |
| | R130Q | | 389G>A | 5033 |
| | T319^{∗} Frameshift mutation | | 955_958del | 4898 |
| | K267Rfs^{∗}9 Base deletion, Frameshift mutation | | 800del | 5809 |
| | N323Mfs^{∗}21 Base deletion, Frameshift mutation | | 968del | 5823 |
| | R173C | | 517C>T | 5089 |
| TP53 | R175H | | 524G>A | 10648 |
| | R248Q | | 743G>A | 10662 |
| | R273H | | 818G>A | 10660 |
| | R273C | | 817C>T | 10659 |
| | R248W | | 742C>T | 10656 |
| | R282W | | 844C>T | 10704 |
| PIK3CA | H1047R | | 3140A>G | 775 |
| | E545K | | 1633G>A | 763 |
| | E542K | | 1624G>A | 760 |
| | H1047L | | 3140A>T | 776 |
| | E545A | | 1634A>C | 12458 |
| RHBDF2 | R192W | | 574C>T | 1735302 |
| | P311Hfs^{∗}26 Frameshift mutation | | 932del | 2743517 |
| | R63S | | 189G>T | 3958782 |
| CYP2E1 | T69M | | 206C>T | 5050047 |
| | F97L | | 289T>C | 4675954 |
| | Q148L | | 443A>T | 6406161 |

In another preferred embodiment, the plurality is at least 2; preferably 2 to 500; preferably 2 to 300; preferably 2 to 200; preferably 2 to 100; preferably 2 to 50 or 2 to 10.

In another preferred embodiment, the tumor is a malignant solid tumor or a hematologic tumor.

In another preferred embodiment, the malignant solid tumor is one or more selected from the group consisting of lung cancer, breast cancer, esophageal cancer, gastric cancer, thyroid cancer, liver cancer, pancreatic cancer, prostate cancer, cervical cancer, colorectal cancer.

As used herein, the term "nucleic acid in red blood cell" or "erythrocyte-derived nucleic acid" refers to nucleic acid obtained from red blood cell/erythrocyte. The present inventors unexpectedly discovered that genetic mutations occurring in cancer cells can be detected in nucleic acid in red blood cells, thus indicating that genetic mutations in cancer cells can be transferred to red blood cells. Based on this, the mutation type of tumor can be identified by detecting tumor-specific gene mutations in red blood cells.

In the present invention, gene sequence of the target nucleic acid in erythrocyte-derived nucleic acid is detected to obtain sequence information of the target nucleic acid, and then, based on the sequence information, mutation type of tumor is determined. The detection of the gene sequence of the target nucleic acid includes sequencing the target nucleic acid using sequencing technology to obtain the nucleotide sequence of the target nucleic acid; and, specifically detecting the gene mutation in the target nucleic acid using other means to determine whether a specific gene mutation is present (e.g., by PCR that specifically amplifies the gene mutation site to detect the presence of the gene mutation in the nucleic acid sample). Thus, as used herein, the term "sequence information" may refer to "nucleotide sequence" or to "presence or absence of a specific genetic mutation". Methods for detection of gene sequences of target nucleic acids include, but are not limited to, high-throughput sequencing techniques, PCR techniques, crisper/cas12 techniques, immunofluorescence assays, etc.

The high-throughput sequencing technology described in the present invention may be any of the high-throughput sequencing technologies currently known in the art.

The term "high-throughput sequencing" is also known as "next-generation" sequencing technology (abbreviated as NGS), is marked by the ability to sequence hundreds of thousands to millions of DNA molecules in parallel at a time and generally by short read lengths. The main types are as follows: Massively Parallel Signature Sequencing (MPSS), Polony Sequencing, 454 pyrosequencing, Illumina (Solexa) sequencing, ABI SOLiD sequencing, Ion semiconductor sequencing, DNA nanoball sequencing, etc.

The PCR techniques described in the present invention include any of the PCR techniques currently known in the art, such as RT-PCR, qRT-PCR, ddPCR, etc.

PCR is an *in vitro* DNA amplification technique in which in the presence of template DNA, primers and four deoxyribonucleotides, by relying on the enzymatic reaction of DNA polymerase, the DNA fragment to be amplified and its complementary oligonucleotide strand primers on both sides are subjected to a three-step cycle of "high temperature denaturation - low temperature annealing - primer extension", resulting in an exponential increase in the number of DNA fragments, thus obtaining the large number of specific gene fragments we need in a short period of time. PCR techniques are often used in combination with other techniques, such as RT-PCR, qRT-PCR, digital PCR, gradient PCR, multiplex PCR, Touchdown PCR, nested PCR, etc.

The crisper/cas12 technology described herein can be any of the specific methods or steps of operation currently known in the art.

The immunofluorescence method described herein can be any of the specific methods or steps of operation currently known in the art.

In the present invention, the terms "a", "an", "this" and "the" refer not only to a singular number of individuals, but include a common category that can be used to illustrate a particular embodiment.

In the present invention, the term "subject to be tested" or "subject" refers to an animal, in particular a mammal, such as a human (including a tumor-bearing patient).

In the present invention, the terms "cancer" and "malignancy" are used interchangeably.

In the present invention, the term "cancer patient" refers to a person or other animal suffering from the cancer described in the present invention.

In the present invention, the term "control" is a normal person (or referred to as a healthy person) or a non-cancer patient.

A normal person (or referred to as a healthy person) is defined as a subject who is not suffering from a disease.

The names of genes listed herein, such as EGFR, have meanings that are well known in the art and should be understood in their broadest sense and are not limited to specific nucleotide sequences. For example, the cDNA sequence of human EGFR, it is understood by those skilled in the art that it is possible for the gene to have one or several nucleotide substitutions, additions or deletions in different subjects due to, for example, genetic variation, and to have essentially the same function as the wild-type gene. These variant genes are still considered to be EGFR genes.

In the method of the present invention, one or more tumor-specific gene mutations, such as 1, 2, 3, 4, 5, 6, 7, 8, 9, 10 or more, can be detected simultaneously. It will be appreciated by those skilled in the art that simultaneous detection of multiple malignancy-specific gene mutations can help to further improve diagnostic accuracy.

Unless otherwise stated, the scientific and technical terms used in this specification shall have the meaning normally understood by a person of ordinary skill in the art. Generally, the nomenclature and techniques used in this specification relating to cell and tissue culture, molecular biology, immunology, microbiology, genetics, and protein and nucleic acid chemistry are well known and commonly used in the art.

Unless otherwise stated, the methods and techniques used in this specification are generally based on methods known and conventional in the art and described in the various references set forth or cited in this specification.

### Identification of tumorigenesis and/or mutation type of tumor

The kit according to the present invention can be used to detect one or more tumor-specific gene mutations in red blood cells, which in turn can be used for positive differential diagnosis of tumor and molecular classification of tumor based on the presence or absence of that particular gene mutation.

Usually, for patients with diagnosed tumors, clinical testing for the mutation type of tumor is required to guide the clinical use of drugs. Thus, the mutation type of tumor can be detected using the kit and method of the present invention with extremely high sensitivity and accuracy.

Nucleic acid within red blood cell as described in the present invention include any form of RNA (e.g., mRNA, MicroRNA, etc.) and/or DNA (e.g., cDNA, circular DNA, etc.) in the red blood cell, which are also referred to in the present invention as erythrocyte-derived nucleic acid.

The nucleic acid may be RNA (e.g., mRNA, etc.) and/or DNA corresponding to any one or more genes of the prior art.

In another preferred embodiment, the nucleic acid comprises a tumor-specific mutation.

In another preferred embodiment, the nucleic acid is derived from a tumor cell.

### Nucleic acid detection reagent set and kit

The nucleic acid detection reagent set for detecting target nucleic acid in red blood cell according to the present invention may include a red blood cell isolation reagent, a nucleic acid extraction reagent, and the like. The red blood cell isolation reagent is a regent used to isolate red blood cells from whole blood, including but not limited to PBS buffer, Ficoll lymphocyte isolation buffer. The nucleic acid extraction reagent includes, but is not limited to, red blood cell lysis buffer, and DNA or RNA extraction reagents that are conventional in the art.

The kit for detecting target nucleic acid in red blood cell according to the present invention comprises the nucleic acid detection reagent set described above, wherein each reagent in the nucleic acid detection reagent set is distributed in the kit in a spatially compartmentalizable manner. The kit also includes an instruction documenting the use of the kit of the present invention for tumor diagnosis, and, the steps for preparing a nucleic acid sample from red blood cells using the nucleic acid detection kit.

The main advantages of the present invention comprise:
Use of nucleic acid (e.g., RNA and/or DNA) derived from tumor tissues in red blood cells as the detection target has very great advantages, including the advantages of easy sampling, simple operation, low cost of detection, and high sensitivity and accuracy in targeting tumor mutations.

The present invention is further described below in connection with specific embodiments. It should be understood that these examples are intended to illustrate the invention only and are not intended to limit the scope of the invention. Experimental methods for which specific conditions are not indicated in the following examples usually follow conventional conditions, or follow the conditions recommended by the manufacturer. Percentages and parts are percentages by weight and parts by weight unless otherwise stated. The experimental materials and reagents used in the following embodiments are commercially available unless otherwise specified.

### Reagents:

RNAse inhibitor (Takara, Cat. No. 2313B(A×5))
Trizol (Invitrogen, Cat. No. 15596026)

### Equipment:

Centrifuge (Thermo Fisher, Cat. No. 75007204)
PCR instrument (ABI 7300 real-time fluorescence quantitative PCR instrument, 7300 real-time PCR system)
High-throughput sequencing instrument (Illumina, HiSeq 4000)

### Example 1 - Preparation of Nucleic acid sample derived from red blood cell (RBC)

### I. Reagents

PBS buffer (number of samples*4 ml): PBS buffer can be prepared and used on site (DEPC water preparation) (Gibco PBS, from Thermo Scientific, Cat. No. 10010023, capacity 500 mL) (PBS buffer, purchased from BOSTER Biological Technology Co., Ltd., Cat. No. AR0030)
Ficoll lymphocyte isolation buffer (sample number*3 ml), (Human lymphocyte isolation buffer, purchased from Dakewe Biotech Co. Ltd., Trade name: DaYou, Cat. No. 7111011/711101X, capacity 100 mL)
DEPC Water
Dry ice

### II. Instruments and consumables

Small high-speed centrifuge for cryogenic centrifugation (1.5 ml Eppendorf centrifugation)
Thermo Scientific Heraeus Multifuge X1R Cryogenic High Speed Centrifuge (15 ml Centrifugal tubes)
Full set of pipettes (Eppendorf) 1000 µl/200 µl/100 µl/20 µl/10 µl/2.5 µl
Full set of pipette tips (DNA/RNase free) 1000 µl/200 µl/10 µl/
15 ml centrifuge tubes (= number of samples), 1.5 ml Eppendorf (= 5*number of samples, 3 RBCs, 2 sera) (DNA/RNase free)
Eppendorf rack, 15 ml centrifuge tube rack, 1.5 ml Eppendorf storage box, sample delivery bag, foam box, sealing film, gloves, and marker

### III. Experimental operation

Whole blood separation steps:
① Placed the anticoagulated tubes of stored whole blood in a centrifuge at 4°C, 150 g, for 10 minutes, immediately after end of centrifugation, opened the centrifuge to bring it back to room temperature.
② Aspirated and discarded the centrifuged supernatant with a 1 ml pipette.
③ Added 2 ml of PBS to the RBC precipitate and mixed with a pipette by gently blowing to dilute the RBC.
④ Then tilted the 15 ml centrifuge tube added with 3 ml Ficoll lymphocyte isolation buffer and gently added diluted RBCs along the wall of the tube to the upper layer of the lymphocyte isolate surface (handled gently to avoid RBCs sinking to the bottom).
⑤ 800 g, 25°C at room temperature, centrifuged for 15 minutes.
⑥ Discarded all supernatants above the RBCs with a 1 mL pipette, using gentle movements to prevent other nucleated cells from falling to the RBC layer.
⑦ Added an equal volume of PBS (1.5-2 ml) to the RBC precipitate, and mixed gently with a pipette to dilute the RBC, and waited until the deep red on the wall of the tube tip was all blown into bright red to indicate that it had been mixed
⑧ 150 g, 25°C room temperature, centrifuged 15 minutes, total 20 minutes
⑨ Discarded the supernatant with a pipette, and the lower precipitate was the pure RBC, dispensed the RBC into a 1.5 ml RNase-free Eppendorf, which was the RBC sample, and stored it at -80°C in the refrigerator for later use.

An appropriate amount of red blood cell lysis buffer was added to the RBC sample (only swollen and ruptured erythrocytes, while not other cells), centrifuged again, and the supernatant was taken as pure erythrocyte inclusions, and further extracted with DNA or RNA extraction kit (DNA extraction kit such as QIAamp Circulating Nucleic Acid Kit from QIAGEN; RNA extraction kit such as High Performance Blood Total RNA Extraction Kit (DP443) from Tiangen Biotech (Beijing) Co. Ltd.) according to the kit instruction to obtain a nucleic acid sample derived from red blood cell.

### Example 2 - Detection of Lung Cancer

Using the method and kit of the present invention, 28 patients were tested for tumor-specific mutations, including clinically confirmed 23 cases of lung adenocarcinoma, 2 cases of inflammatory pseudotumor, 2 cases of lung squamous carcinoma, and 1 case of thymoma.

Red blood cell (RBC)-derived nucleic acid samples were prepared by referring to Example 1.

Tissue-derived nucleic acid samples were prepared by methods that are conventional in the art, for example, they can be prepared using a kit (GeneReadTM DNA FFPE Kit, Qiagen).

Serum-derived nucleic acid samples were prepared by methods that are conventional in the art, for example, they can be prepared using a kit (QIAamp Circulating Nucleic Acid Kit, Qiagen).

The tissue-derived nucleic acid samples prepared above were subjected to EGFR gene mutation analysis by NGS sequencing (high-throughput sequencing instrument (Illumina, HiSeq4000)). Enrichment and amplification of target genes were conducted using Geneseeq hybridization enrichment probes (Nanjing GENESEEQ Technology Inc.) for established DNA libraries, including: hybridization of DNA capture probes with libraries; cleaning and recovery of capture library products; binding of capture libraries to streptavidin magnetic beads; cleaning of magnetic bead capture libraries to remove non-specific binding libraries. The captured library was sampled on the Illumina HiSeq 4000 high-throughput sequencing platform according to the operating steps in the Illumina kit instruction. DNA was formed into DNA clusters on the kit Flow cell, and the sequencing platform completed DNA high-throughput sequencing through a cycle of individual base synthesis followed by pause, fluorescence detection, and synthesis recovery.

The red blood cell (RBC)-derived nucleic acid samples were subjected to NGS sequencing and qPCR assay, respectively, where NGS sequencing was performed as described above. qPCR assay was performed using Therascreen EGFR RGQ PCR kit or Therascreen KRAS RGQ PCR kit (QIAGEN).

The serum-derived nucleic acid samples were subjected to NGS sequencing of serum ctDNA (high-throughput sequencing instrument (Illumina, HiSeq 4000)).

The detection results are shown in the table below. The results showed that tumor-specific mutation detection using NGS sequencing was 100% consistent for the RBC-derived nucleic acid samples and for the tumor tissue-derived nucleic acid samples, and no mutations were detected in all non-tumor samples. In contrast, NGS sequencing assays using serum ctDNA detected only 9% (1/11) of tumor-specific mutations. Therefore, the RBC-derived nucleic acid samples of the present invention are fully capable of replacing tissue-derived nucleic acid samples for use as nucleic acid samples for tumor diagnosis, and have the advantages of high accuracy, high sensitivity, easy sampling, and better safety.

In addition, the RBC-derived nucleic acid samples were tested using qPCR, and tumor-specific mutations were accurately detected in all of the 10 positive samples tested. It showed that the RBC-derived nucleic acid samples prepared using the method of the present invention were of good quality and can also be used for tumor diagnosis by qPCR method, thus further reducing the cost of tumor diagnosis.

**Table 1**

| Sample No. | Type | Stage of an illness | Tissue mutation type (NGS) | Erythrocyte DNA detection results (NGS) | Erythrocyte DNA detection results (qPCR) | Serum ctDNA detection results (NGS) |
|---|---|---|---|---|---|---|
| GL-1 | lung adenocarcinoma | IA(T1N0M0) | EGFR-19del | EGFR-19del | EGFR-19del | - |
| GL-3 | lung adenocarcinoma | IA(T1N0M0) | EGFR-19del | EGFR-19del | EGFR-19del | - |
| GL-4 | lung adenocarcinoma | IIIA(T2N2M0) | EGFR-L858R | EGFR-L858R | N | EGFR-L858R |
| GL-5 | lung adenocarcinoma | IA(T1N0M0) | - | - | N | - |
| GL-6 | Inflammatory pseudotumor | NA | - | - | N | - |
| GL-7 | lung adenocarcinoma | IA(T1N0M0) | - | - | - | - |
| GL-9 | lung adenocarcinoma | IB(T2N0M0) | - | - | N | - |
| GL-10 | Inflammatory pseudotumor | NA | - | - | N | - |
| GL-13 | Lung squamous carcinoma | IIB(13NOMO) | - | - | - | - |
| GL-14 | lung adenocarcinoma | IA(T1N0M0) | - | - | N | - |
| XH-1 | lung adenocarcinoma | IB(T2N0M0) | - | - | N | - |
| XH-2 | Thymoma | NA | - | - | N | - |
| XH-3 | lung adenocarcinoma | IA(T1N0M0) | - | - | N | - |
| XH-4 | lung adenocarcinoma | IA(T1N0M0) | - | - | N | - |
| XH-6 | lung adenocarcinoma | IA(T1N0M0) | - | - | N | - |
| XH-8 | lung adenocarcinoma | IA(T1N0M0) | EGFR-L858R | EGFR-L858R | EGFR-L858R | - |
| XH-9 | Lung squamous carcinoma | IB(T1N0M0) | EGFR-L858R | EGFR-L858R | EGFR-L858R | - |
| XH-10 | lung adenocarcinoma | IA(T1N0M0) | EGFR-L858R | EGFR-L858R | EGFR-L858R | - |
| XH-11 | lung adenocarcinoma | IB(T2N0M0) | EGFR-L858R | EGFR-L858R | EGFR-L858R | - |
| XH-12 | lung adenocarcinoma | IA(T1N0M0) | - | - | N | - |
| XH-13 | lung adenocarcinoma | IA(T1N0M0) | - | - | N | - |
| XH-14 | lung adenocarcinoma | IA(T1N0M0) | EGFR-19del | EGFR-19de1 | EGFR-19de1 | - |
| XH-20 | lung adenocarcinoma | IA(T1N0M0) | EGFR-L858R | EGFR-L858R | EGFR-L858R | - |
| XH-21 | lung adenocarcinoma | IA(T1N0M0) | EGFR-L858R | EGFR-L858R | EGFR-L858R | - |
| XH-22 | lung adenocarcinoma | IA(T1N0M0) | EGFR-19del | EGFR-19de1 | EGFR-19de1 | - |
| XH-24 | lung adenocarcinoma | IA(T1N0M0) | - | - | N | - |
| XH-25 | lung adenocarcinoma | IA(T1N0M0) | - | - | N | - |
| XH-26 | lung adenocarcinoma | IA(T1N0M0) | - | - | N | - |

| | | | | | | |
|---|---|---|---|---|---|---|
| Note: "NA" is no staging information; "-" is no mutation detected, "N" is no detection. | | | | | | |

Existing techniques have shown that with tDNA (tumor tissue) as the gold standard control, the sensitivity and concordance rate of EGFR tumor mutation detection using ctDNA (blood sample) (detection platform: Ion second-generation sequencer (Life Technologies)) are low, which is difficult to meet the clinical diagnostic needs. The kit and method of the present invention, with RBC-derived nucleic acid samples, greatly improved the sensitivity and consistency of tumor mutation detection and achieved unanticipated technical results.

### Example 3 - Detection of Colorectal tumor sample

Tumor-specific mutation detection was performed on blood samples from postoperative colorectal tumor patients (including some patients undergoing colonoscopy and biopsy), and tumor-specific mutation detection was also performed on blood samples from the same patients using the common ctDNA method, and the results of mutation detection were compared with those of their tumor tissue samples, respectively. These included clinically confirmed 13 cases of colon cancer, 5 cases of rectal cancer, and 7 cases of benign lesions (including colonic adenomas, inflammatory bowel polyps, and saprophytic polyps).

Referring to Example 2 for specific detection procedures, the detection results are shown in Table 2 below. The results showed that the results of colorectal tumor-specific mutation detection using NGS sequencing were 100% consistent for the RBC-derived nucleic acid samples and for the tumor tissue-derived nucleic acid samples, and no mutations were detected in all non-tumor samples. Therefore, the RBC-derived nucleic acid samples of the present invention are fully capable of replacing tissue-derived nucleic acid samples for use as nucleic acid samples for tumor diagnosis, and have the advantages of high accuracy, high sensitivity, easy sampling, and better safety.

In addition, the RBC-derived nucleic acid samples were tested using qPCR, and tumor-specific mutations were accurately detected in all of the 7 positive samples tested. It showed that the RBC-derived nucleic acid samples prepared using the method of the present invention were of good quality and can also be used for tumor diagnosis by qPCR method, thus further reducing the cost of tumor diagnosis.

**Table 2**

| Sample No. | Type | Stage of an illness | Tissue mutation type (NGS) | Erythrocyte DNA detection results (NGS) | Erythrocyte DNA detection results (qPCR) | Serum ctDNA detection results (NGS) |
|---|---|---|---|---|---|---|
| CL-1 | colon cancer | Stage I | KRAS-G120D | KRAS-G120D | KRAS-G120D | - |
| CL-2 | colonic adenoma | NA | - | - | N | N |
| CL-3 | rectal cancer | Stage II | NRAS-Q61K | NRAS-Q61K | NRAS-Q61K | - |
| CL-4 | colon cancer | Stage II | KRAS-G120D | KRAS-G120D | N | - |
| CL-5 | colon cancer | Stage I | - | - | - | N |
| CL-6 | rectal cancer | Stage III | KRAS-G120D | KRAS-G120D | KRAS-G120D | KRAS-G120D |
| CL-7 | rectal cancer | Stage II | NRAS-Q61K | NRAS-Q61K | NRAS-Q61K | - |
| CL-8 | metaplastic intestinal polyps | NA | - | - | N | N |
| CL-9 | colon cancer | Stage II | - | - | N | - |
| CL-10 | colon cancer | Stage I | BRAF-V600E | BRAF-V600E | BRAF-V600E | - |
| CL-11 | colon cancer | Stage III | - | -- | - | - |
| CL-12 | colon cancer | Stage II | - | - | N | - |
| CL-13 | colon cancer | Stage II | - | - | N | - |
| CL-14 | rectal cancer | Stage III | - | - | N | - |
| CL-15 | colon cancer | Stage I | NRAS-Q61R | NRAS-Q61R | NRAS-Q61R | - |
| CL-16 | intestinal polyps | NA | - | - | N | N |
| CL-17 | colon cancer | Stage II | - | - | N | - |
| CL-18 | inflammatory polyps | NA | - | - | N | N |
| CL-19 | rectal cancer | Stage III | KRAS-G120D | KRAS-G120D | KRAS-G120D | KRAS-G120D |
| CL-20 | colon cancer | Stage II | - | - | - | - |
| CL-21 | colonic adenoma | NA | - | - | N | N |
| CL-22 | colon cancer | Stage III | - | - | N | - |
| CL-23 | inflammatory polyps | NA | - | - | N | N |
| CL-24 | colonic adenoma | NA | - | - | N | - |
| CL-25 | colon cancer | Stage III | - | - | N | - |

| | | | | | | |
|---|---|---|---|---|---|---|
| Note: "NA" is no staging information; "-" is no mutation detected, "N" is no detection. | | | | | | |

### Example 4 - Detection of Breast tumor sample

Tumor-specific mutation detection was performed on blood samples from postoperative breast tumor patients (including patients with hollow needle puncture), and tumor-specific mutation detection was also performed on blood samples from the same patients using the common ctDNA method, and the results of mutation detection were compared with those of their tumor tissue samples, respectively. These included clinically confirmed 19 cases of breast cancer and 6 cases of benign lesions (including breast fibroadenoma and cystic breast hyperplasia).

Referring to Example 2 for specific detection procedures, the detection results are shown in Table 3 below. The results showed that the results of breast tumor-specific mutation detection using NGS sequencing were 100% consistent for the RBC-derived nucleic acid samples and for the tumor tissue-derived nucleic acid samples. Therefore, the RBC-derived nucleic acid samples of the present invention are fully capable of replacing tissue-derived nucleic acid samples for use as nucleic acid samples for tumor diagnosis, and have the advantages of high accuracy, high sensitivity, easy sampling, and better safety.

In addition, the RBC-derived nucleic acid samples were tested using qPCR, and tumor-specific mutations were accurately detected in all of the 9 positive samples tested. It showed that the RBC-derived nucleic acid samples prepared using the method of the present invention were of good quality and can also be used for tumor diagnosis by qPCR method, thus further reducing the cost of tumor diagnosis.

**Table 3**

| Sample No. | Type | Stage of an illness | Tissue mutation type (NGS) | Erythrocyte DNA detection results (NGS) | Erythrocyte DNA detection results (qPCR) | Serum ctDNA detection results (NGS) |
|---|---|---|---|---|---|---|
| BL-1 | breast cancer | Stage II | TP53-R273C | TP53-R273C | TP53-R273C | - |
| BL-2 | breast cancer | Stage I | - | - | N | N |
| BL-3 | breast cancer | Stage I | - | - | N | - |
| BL-4 | breast cancer | Stage II | BRAC2-N372H | BRAC2-N372H | BRAC2-N372H | - |
| BL-5 | breast cancer | Stage II | PTEN-R130G | PTEN-R130G | PTEN-R130G | - |
| BL-6 | breast cancer | Stage III | BRAC2-N372H | BRAC2-N372H | BRAC2-N372H | BRAC2-N372H |
| BL-7 | breast fibroadenoma | NA | - | - | N | N |
| BL-8 | breast cancer | Stage I | - | - | - | - |
| BL-9 | breast cancer | Stage II | - | - | N | - |
| BL-10 | breast cancer | Stage III | - | - | - | - |
| BL-11 | breast cancer | Stage II | - | - | N | - |
| BL-12 | cystic breast hyperplasia | NA | - | - | N | N |
| BL-13 | breast cancer | Stage III | - | - | - | N |
| BL-14 | breast cancer | Stage I | BRAC1-H619N | BRAC1-H619N | BRAC1-H619N | - |
| BL-15 | breast cancer | Stage IV | - | - | - | - |
| BL-16 | breast fibroadenoma | NA | - | - | N | N |
| BL-17 | breast cancer | Stage II | - | - | - | - |
| BL-18 | breast cancer | Stage II | - | - | N | N |
| BL-19 | breast cancer | Stage III | - | - | N | - |
| BL-20 | breast fibroadenoma | NA | - | - | N | N |
| BL-21 | breast cancer | Stage II | BRAC1-H619N | BRAC1-H619N | BRAC1-H619N | - |
| BL-22 | breast fibroadenoma | NA | - | - | N | N |
| BL-23 | cystic breast hyperplasia | NA | - | - | N | N |
| BL-24 | breast cancer | Stage II | BRAC1-H619N | BRAC1-H619N | N | N |
| BL-25 | breast cancer | Stage II | BRAC2-N372H | BRAC2-N372H | N | N |

| | | | | | | |
|---|---|---|---|---|---|---|
| Note: "NA" is no staging information; "-" is no mutation detected, "N" is no detection. | | | | | | |

### Example 5 - Detection of Esophageal tumor sample

Tumor-specific mutation detection was performed on blood samples from postoperative esophageal tumor patients (including some patients undergoing esophagoscopic surgery and biopsy), and tumor-specific mutation detection was also performed on blood samples from the same patients using the common ctDNA method, and the results of mutation detection were compared with those of their tumor tissue samples, respectively. These included clinically confirmed 12 cases of esophageal epithelial carcinoma, 3 cases of cardia carcinoma, 1 case of esophageal adenocarcinoma, and 2 cases of esophageal leiomyoma (benign).

Referring to Example 2 for specific detection procedures, the detection results are shown in Table 4 below. The results showed that the results of esophageal tumor-specific mutation detection using NGS sequencing were 100% consistent for the RBC-derived nucleic acid samples and for the tumor tissue-derived nucleic acid samples, and no mutations were detected in all non-tumor samples. Therefore, the RBC-derived nucleic acid samples of the present invention are fully capable of replacing tissue-derived nucleic acid samples for use as nucleic acid samples for tumor diagnosis, and have the advantages of high accuracy, high sensitivity, easy sampling, and better safety.

In addition, the RBC-derived nucleic acid samples were tested using qPCR, and tumor-specific mutations were accurately detected. It showed that the RBC-derived nucleic acid samples prepared using the method of the present invention were of good quality and can also be used for tumor diagnosis by qPCR method, thus further reducing the cost of tumor diagnosis.

**Table 4**

| Sample No. | Type | Stage of an illness | Tissue mutation type (NGS) | Erythrocyte DNA detection results (NGS) | Erythrocyte DNA detection results (qPCR) | Serum ctDNA detection results (NGS) |
|---|---|---|---|---|---|---|
| EG-1 | esophageal epithelial carcinoma | Stage II | - | - | - | - |
| EG-2 | esophageal epithelial carcinoma | Stage II | RHBDF2-R63S | RHBDF2-R63 S | N | - |
| EG-3 | cardia carcinoma | Stage I | - | - | N | - |
| EG-4 | esophageal leiomyoma | NA | - | - | N | N |
| EG-5 | esophageal epithelial carcinoma | Stage III | CYP2E1-T69M | CYP2E1-T69M | CYP2E1-T69M | - |
| EG-6 | esophageal squamous cell carcinoma | Stage II | - | - | - | - |
| EG-7 | esophageal carcinoma | Stage II | RHBDF2-R63S | RHBDF2-R63S | N | - |
| EG-8 | esophageal epithelial carcinoma | Stage I | - | - | N | N |
| EG-9 | esophageal leiomyoma | NA | - | - | N | N |
| EG-10 | esophageal epithelial carcinoma | Stage III | - | - | - | - |
| EG-11 | esophageal epithelial carcinoma | Stage II | - | - | - | - |
| EG-12 | esophageal epithelial carcinoma | Stage II | - | - | N | - |
| EG-13 | cardia cancer | Stage III | TP53-R248Q | TP53-R248Q | N | - |
| EG-14 | esophageal leiomyoma | NA | - | - | - | - |
| EG-15 | esophageal epithelial carcinoma | Stage III | CYP2E1-T69M | CYP2E1-T69M | CYP2E1-T69M | - |
| EG-16 | cardia carcinoma | Stage II | - | - | N | - |
| EG-17 | esophageal adenocarcinoma | Stage II | - | - | N | - |
| EG-18 | esophageal epithelial carcinoma | Stage II | - | - | N | - |

| | | | | | | |
|---|---|---|---|---|---|---|
| Note: "NA" is no staging information; "-" is no mutation detected, "N" is no detection. | | | | | | |

All literature referred to in the present invention are cited by reference in this application as if each piece of literature was cited separately as a reference. It should also be understood that, after reading the above lecture on the invention, a person skilled in the art may make various alterations or modifications to the invention, which in equivalent form likewise fall within the scope defined by the claims appended to this application.

## Claims

1. A kit comprising a nucleic acid detection reagent set which is configured to detect a target nucleic acid in red blood cell, wherein said kit is configured to identify tumorigenesis and/or mutation type of tumor based on result of target nucleic acid detection in red blood cell.

2. The kit according to claim 1, wherein said kit is configured to
(1) be used for positive differential diagnosis of tumor based on identified mutation; or
(2) determine molecular classification of tumor of a subject to be tested based on the identified mutation type of the tumor; or
(3) guide clinical administration of a drug based on identified mutation type of tumor.

3. The kit according to claim 1, wherein said target nucleic acid is extracted from the population to be differentially diagnosed for positive.

4. The kit according to claim 1, wherein identification of tumorigenesis and/or mutation type of tumor comprises identifying a mutation occurring in a gene selected from the group consisting of EGFR, KRAS, NRAS, NTRK, BRAF, HER2, MET, ALK, ROS-1, RET, BRAC1/2, PTEN, TP53, PIK3CA, RHBDF2, BLM, PALB2, CTHRC1, ASCC1, MSR1, ALDH2, ADH1B, CYP2E1, and GSTM1.

5. A nucleic acid sample, wherein said nucleic acid sample is used to identify tumorigenesis and/or mutation type of tumor, and said nucleic acid sample is erythrocyte-derived nucleic acid.

6. A method of preparing a nucleic acid sample, wherein said nucleic acid sample is used to identify tumorigenesis and/or mutation type of tumor, and said method comprises steps of:
(1) isolating red blood cells from whole blood;
(2) extracting nucleic acid from the red blood cells obtained in step (1), thereby producing said nucleic acid sample.

7. Use of a nucleic acid detection set in manufacture of a kit for identifying tumorigenesis and/or mutation type of tumor, wherein said nucleic acid detection set is configured to detect nucleic acid in red blood cell.

8. The use according to claim 7, wherein said nucleic acid detection kit is used to detect nucleic acid in red blood cell, comprising steps of
(a) preparing an erythrocyte-derived nucleic acid sample of a subject to be tested; and
(b) detecting one or more tumor-specific gene mutations in the nucleic acid sample.

9. The use according to claim 8, wherein gene sequence of target nucleic acid is detected in step (b) to obtain sequence information of the target nucleic acid, and then tumorigenesis and/or mutation type of tumor is identified based on the obtained sequence information of the target nucleic acid.

10. The use according to claim 7, wherein said nucleic acid sample is a nucleic acid sample from a tumor-bearing patient.

11. The use according to claim 7, wherein a tumor-specific gene mutation comprises a mutation occurring in a gene selected from the group consisting of EGFR, KRAS, NRAS, NTRK, BRAF, HER2, MET, ALK, ROS-1, RET, BRAC1/2, PTEN, TP53, PIK3CA, RHBDF2, BLM, PALB2, CTHRC1, ASCC1, MSR1, ALDH2, ADH1B, CYP2E1, and GSTM1.

12. A method for identifying tumorigenesis and/or mutation type of tumor, wherein said method comprises steps of:
(a) providing an erythrocyte-derived nucleic acid sample of a subject to be tested; and
(b) detecting one or more tumor-specific gene mutations in the nucleic acid sample.

13. The method according to claim 12, wherein gene sequence of target nucleic acid is detected in step (b) to obtain sequence information of the target nucleic acid, and then tumorigenesis and/or mutation type of tumor is determined based on the sequence information detected in step (b).

14. The method according to claim 12, wherein said nucleic acid sample is a nucleic acid sample from a tumor-bearing patient.

15. The method according to claim 12, wherein the tumor-specific gene mutation comprises a mutation occurring in a gene selected from the group consisting of EGFR, KRAS, NRAS, NTRK, BRAF, HER2, MET, ALK, ROS-1, RET, BRAC1/2, PTEN, TP53, PIK3CA, RHBDF2, BLM, PALB2, CTHRC1, ASCC1, MSR1, ALDH2, ADH1B, CYP2E1, and GSTM1.
